Europäisches Patentamt

European Patent Office

Office européen des brevets

(11) Publication number: **0 263 017 B1**

# EUROPEAN PATENT SPECIFICATION

(45) Date of publication of patent specification: **22.01.92**  (51) Int. Cl.⁵: **A01H 1/02**, C12N 15/00

(21) Application number: **87402105.8**

(22) Date of filing: **21.09.87**

(54) Process for preparing gramineous hybrid plants.

(30) Priority: **20.09.86 JP 223346/86**

(43) Date of publication of application:
**06.04.88 Bulletin 88/14**

(45) Publication of the grant of the patent:
**22.01.92 Bulletin 92/04**

(84) Designated Contracting States:
**DE FR GB**

(56) References cited:
**EP-A- 0 114 529**

**PLANT & CELL PHYSIOL., vol. 20, no. 7, 1979, pages 1441-1443; M. SENDA et al.: "Induction of cell fusion of plant protoplasts by electrical stimulation"**

(73) Proprietor: **MITSUBISHI KASEI CORPORATION**
**5-2, Marunouchi 2-chome Chiyoda-ku**
**Tokyo 100(JP)**

Proprietor: **MITSUBISHI CORPORATION**
**6-3, Marunouchi 2-chome**
**Chiyoda-ku Tokyo 100(JP)**

(72) Inventor: **Shimamoto, Ko B-312 Mitsubishi-Kasei-Apaato**
**3 Sakuradai Midori-ku**
**Yokohama-shi Kanagawa-ken(JP)**
Inventor: **Watanabe-Kyozuka, Junko**
**3-8-17 Seta Setagaya-ku**
**Tokyo(JP)**
Inventor: **Terada, Rie**
**34-4 Shiratoridai Midori-ku**
**Yokohama-shi Kanagawa-ken(JP)**
Inventor: **Hayashi, Yasuyuki Mitsubishi-Kasei-Tachibana-Ryo**
**2-6-3 Tachibanadai Midori-ku**
**Yokohama-shi Kanagawa-ken(JP)**

(74) Representative: **Gutmann, Ernest et al**
**S.C. Ernest Gutmann - Yves Plasseraud 67,**
**boulevard Haussmann**
**F-75008 Paris(FR)**

**Description**

BACKGROUND OF THE INVENTION

Field of the Invention:

The present invention relates to a process for producing hybrid plants in the Gramineae by the cell fusion method.

Description of the Prior Art:

The cell fusion method is an effective means for obtaining a hybrid between different species or genera, which cannot be obtained by conventional breeding methods.

With respect to gramineous plants including the cereals, however, there have been few reports. The cell fusion between pearl millet (Pennisetum glaucum) and guinea grass (Panicum maximum) is described in Mol. Gen. Genet., 203, 365-370 (1986).

European patent application EP-A-114529 discloses a process for preparing gramineous hybrid plants. After isolation of protoplasts from plant cells, cell fusion is carried out by treating the plant protoplasts with a fusing agent. Fusion is promoted by incubating the protoplasts in an incubation medium having an osmotic pressure lower than that required for maintaining the plant protoplasts in a stable form. Production of hybrids of Oryza sativa with another gramineous plant is not described.

Senda et al., in Plant and Cell Physiol. 20, (7), 1441-1443 (1979) describe the induction of cell fusion of plant protoplasts by electrical stimulation. An electrical treatment consisting of the application of direct current between successive alternating current applications is not described.

In order to efficiently produce hybrid plants, conditions for cell fusion, and methods for screening hybrids and plant regeneration should be established. However, there have been few reports on such conditions and methods concerning plants in the Gramineae.

SUMMARY OF THE INVENTION

Therefore, it is an object of the present invention to provide an efficient process for producing such a hybrid plant in the Gramineae by the cell fusion method, more particularly to provide a process for producing a somatic hybrid plant between rice (Oryza sativa) and another gramineous plant.

BRIEF DESCRIPTION OF THE DRAWING

The invention will be described in detail with reference to the attached drawing in which:
Fig. 1 is a picture showing the electrophoretic band pattern of LAP (leucine aminopeptidase) isozymes; Os corresponds to rice (Oryza sativa), Eo to Echinocloa oryzicola, $H_1$ and $H_2$ to hybrid calluses obtained according to the process of the invention, and Mix to a mixture of Os and Eo; and
Fig. 2 is a picture showing the electrophoretic band pattern of ADH (alcohol dehydrogenase) isozymes; OS corresponds to rice (Oryza sativa), OP to Oryza perrieri, $H_1$ and $H_2$ to hybrid plants obtained according to the process of the invention, and MIX to a mixture of OS and OP.

DETAILED DESCRIPTION OF THE INVENTION

The above mentioned objects can be attained by the practice of the process according to the present invention, in which protoplasts preprared from callus or suspension cells derived from rice (Oryza sativa) and protoplasts prepared from callus or suspension cells derived from another Gramineae species are subjected to cell fusion, by an electric treatment, said electric treatment comprising the application of direct current between successive alternating current applications, the resulting fused cells are cultured in a culture medium containing cultured cells of rice (Oryza sativa) as nurse cells to thereby form colonies, and the colonies are further cultured in a medium containing plant hormones.

Rice (Oryza sativa) plants which can be used in the practice of the invention may include many rice varieties such as NIHONBARE, KOSHIHIKARI, SASANISHIKI and the like.

Suspension cells or callus derived from mature or immature seeds, leaf sheaths or root tissues, or the like, of rice is cultivated in a liquid medium to prepare protoplasts.

More specifically, for instance, the above mentioned plant material may be sterilized by sodium hypochlorite or the like, placed on e.g. Murashige and Skoog (MS) agar culture medium (Physiol. Plant, 15, 473-497 (1962)), and then cultured at 25-28° C under 2,000-3 300 lux (17 hr/day). The thus induced callus may then be subjected to shake culture at 50-150 rpm under the same temperature and light conditions as mentioned above in MS or R2 liquid culture medium (Plant Cell Physiol., 14, 1113-1121 (1973)).

In the preparation of protoplasts of another gramineous plant other than said rice plant, for example, Echinocloa oryzicola, or wild Oryza species, Oryza officinalis, Oryza eichingeri, Oryza brachyantha, Oryza perrieri, etc., mature seeds, or leaf sheaths or roots of a seedling grown under aseptic conditions may be placed on MS agar medium and cultivated. The resulting callus may be subjected to shake culture in a liquid medium under similar conditions as above mentioned.

Each of the thus cultured cells is treated with a cell wall degrading enzyme such as cellulase, macerozyme or the like in an enzymic solution containing the enzymes at 25-30° C and 0-50 spm (stroke per minute) for about 3-16 hours to prepare corresponding protoplasts from each of said cultured cells. After the enzyme treatment, filtration may be effected to remove undigested materials, and KMC solution (0.118 M KCl, 0.0817 M MgCl$_2$ and 0.085 M CaCl$_2$, pH 6.0: see Theor. Appl. Genet., 53, 57-63 (1978)), may be added to the filtrate in an amount of 2 to 5 times by volume of the filtrate. The resulting mixture may be centrifuged to produce purified protoplasts.

The resulting protoplasts are suspended in a solution containing mannitol, morpholinoethanesulphonic acid (MES), etc., and then subjected to cell fusion by electric treatment with alternate and direct currents.

It is preferred to preliminarily treat the protoplasts from rice (Oryza sativa) with an iodine compound, such as iodoacetamide, monoiodoacetic acid or the like, of a concentration of 1-50 mM near 4° C for 5-30 minutes.

On the other hand, the protoplasts from another gramineous plant may optionally be subjected to preliminary treatment with a Rhodamine compound, such as Rhodamine 6G or the like, of a concentration of 5-100 $\mu$M at room temperature for about 10-120 minutes. In such treatment, the density of the protoplast may preferably be $1 \times 10^5$ to $1 \times 10^7$ cells per ml. Such treatment renders the protoplasts incapable of dividing by themselves in a culture medium for cultivating fused cells as will described hereinbelow. If the protoplast from another gramineous plant other than rice has no capacity of division in the culture medium described below, such treatment as described above with a Rhodamine compound may not necessarily be effected.

In the cell fusion of the protoplasts by electric treatment, both the protoplasts from rice and the protoplasts from another gramineous plant are mixed in a solution containing 0.2-0.6 M mannitol and 0.05-0.5% MES so that the cell densities of the two protoplasts are equal to each other and the total cell density is $1-3 \times 10^7$ per ml; an alternate current of 2,000-7,000 KHz and 50-500 V/cm is applied to the mixture for 1 to 30 seconds; 1 to 10 pulses of direct current with 1.5-7.5 KV/cm are then applied with a duration of each pulse of 1-100 microseconds at an interval between two successive pulses of 0.1 to 5 seconds; and thereafter, the above mentioned alternate current is again applied for 1 to 200 seconds.

Preferably, the thus treated cells may be suspended in e.g. R2/MS liquid medium containing the inorganic components of R2 and the vitamines of MS, or MS medium, preferably containing 0.2-0.5% potassium nitrate as a nitrogen source; the suspension may then be mixed with an equal amount of R2/MS medium or MS medium containing about 1.0-3.0% agarose; and the resulting mixture may be quickly spread in a petri dish to solidify it into a thin gel film. The cell density in the gel may be about $2-8 \times 10^6$ per ml and the thickness of the agarose film may preferably be approximately 0.7 mm on the average.

The solidified agarose gel may then be cut into pieces having a size of about 5 to 20 mm and cultivated in the aforementioned liquid culture medium. This cultivation may be effected under the dark at 23-27° C with slow shaking (20-50 rpm) in the presence of cultured cells of rice plant (Oryza sativa) in an amount of about 100-300 mg per fresh weight per petri dish.

Alternatively, such cultured cells of rice may be placed in a vessel equipped with a membrane filter or the like at the bottom. This vessel may be immersed into the liquid medium containing the above treated cells to allow the exchange of nutrients through the filter.

Preferably, the cultured cells of rice used in the invention may essentially consist of small cell aggregates showing active cell division. Such cultured cells may be easily obtained according to known methods: for example, a callus obtained from a seed, stem, root or anther of rice plant may be sub-cultured in a liquid medium to select desirable cells.

During such culture of the agarose gel as described above, only the properly fused cells can proliferate.

After 3 to 7 day culture the first cell division will be observed, and by about 10th day, all the cells having the division capacity will have finished their first cell division. These cells can now proliferate without co-existence of cultured rice cells. Then, the culture medium is replaced with a new medium while at the same time the cultured rice cells are removed out. In 3 to 5 week culture colonies of about 0.5 to 1.0 mm in diameter will be formed.

These colonies are then cultivated in a growth medium, for example, an agar medium comprising $N_6$ basal medium (Sci. Sin., 16, 659-688 (1975)) to which about 2 mg/l of a plant hormone, e.g., 2,4-dichlorophenoxyacetic acid (2,4-D) and 0.1-1.0% agarose are added, under light of 1,000-4,000 lux at 23-27°C for 2 to 4 weeks. Thus, a hybrid callus of 3 to 6 mm in diameter is produced.

The hybrid callus is cultivated in, e.g., $N_6$ medium containing 0.5-1.5% agarose, which is hormone-free or contains 1-10 mg/l cytokinin, under 2,000-4,000 lux at 23-27°C. The formation of somatic embryo will be observed in 2 to 10 weeks. Further cultivation in a hormone-free $N_6$ medium or the like for additional s to 3 weeks will give transplantable hybrid plants.

DESCRIPTION OF THE PREFERRED EMBODIMENTS

The present invention will be more fully described by the following examples. These examples are given by way of illustration only and not intended to limit the scope of the invention defined in attached claims.

EXAMPLE 1:

(1) Preparation of Protoplasts:

A mature seed of a rice variety (Oryza sativa) NIHONBARE was sterilized, placed on MS agar medium (2 mg/l 2,4-D, 8 g/l Bactoagar, pH 5.8), and cultured for 3 weeks at 26°C under 3,000 lux to induce a callus. The callus was then cultivated with shaking in R2/MS liquid medium (inorganic salts of R2, vitamines of MS, 1 mg/l 2,4-D, 30 g/l sucrose, pH 5.8) for several weeks at 120 rpm, 25°C under 3,000 lux. The cultured callus was treated in an enzymic solution containing 4% Cellulase RS, 1% Macerozyme R-10 and 0.4 M mannitol (pH 5.6) at 30°C for 3 to 4 hours. The enzymatically treated materials were then filtered. Four times by volume, based on the volume of the resulting filtrate, of KMC solution (0.118 M KCl, 0.085 M $CaCl_2$, 0.0817 M $MgCl_2$, pH 6.0) was added to the filtrate. The resulting mixture was centrifuged and the precipitated protoplasts were collected and further washed twice with the KMC solution.

On the other hand, a seed of Echinocloa oryzicola was sterilized and incubated on hormone-free MS medium (30 g/l sucrose, 8 g/l Bactoagar, pH 5.8) at 25°C under 3,000 lux to induce germination. Ten days later, leaf sheaths of the plant were cut into disks of 0.5 mm in thickness. These disks were placed on the aforementioned agar medium containing 4 mg/l 2,4-D and cultured for 3 weeks at 25°C under 3,000 lux to induce a callus. The callus was then cultured with shaking in MS liquid medium (1 mg/l 2,4-D, 30 g/l sucrose, pH 5.8) for several weeks at 120 rpm, 25°C under 3,000 lux. The cultured cells were treated in an enzymic solution containing 4% Cellulase RS, 1% Macerozyme R-10 and 0.4 M mannitol (pH 5.6) at 30°C for 3 to 4 hours. After filtration of the thus treated materials, four times by volume, based on the volume of the filtrate, of the KMC solution was added to the filtrate. The resulting mixture was centrifuged and the precipitated protoplasts were collected and further washed twice with the KMC solution.

(2) Iodoacetamide Treatment:

The protoplasts, $6 \times 10^6$ cells, prepared in (1) above from the cultured cells of the rice variety, were suspended in KMC solution (5 ml). A 50 mM iodoacetamide solution was added to the suspension so that the final concentration was 25 mM and incubated under the dark at 4°C for 15 minutes. After the treatment, the materials were centrifuged for 5 minutes and the collected protoplasts were twice washed with KMC solution.

(3) Cell Fusion:

The protoplasts, prepared from Echinocloa oryzicola and Oryza sativa in (1) and (2) above, respectively, were suspended in a solution containing 0.45 M mannitol and 1 g/l MES (pH 5.6) so that the cell density of each protoplast was $1-2 \times 10^7$ per ml. The suspension (100 to 200 $\mu$l) was subjected to electric treatment by GCA1000Z cell fusion apparatus. The fusion was effected by first applying an alternate current of 4,000 KHz, 250 V/cm for 6 to 7 seconds, then a direct current of 3.5 KV/cm for 10 microseconds three times with an interval of 1 second, and again an alternate current of 4,000 KHz while gradually reducing the voltage from 250 V/cm over 30 seconds. The thus fused cells were directly cultivated in the following steps.

(4) Culture of the Fused Cells:

The fused cells prepared in (3) above were suspended in R2/MS medium (2 mg/l 2,4-D) 0.4 M sucrose, pH 5.6) containing 0.4% KNO$_3$. This suspension (0.5 ml) was mixed with 0.5 ml of R2/MS medium containing 2% agarose which had been warmed to about 40°C, and the mixture was quickly and uniformly spread on a petri dish of 35 mm in diameter to solidify. The cell density was then $5 \times 10^6$ per ml.

The solidified agarose gel was cut into pieces of about 5 mm $\times$ 20 mm. The pieces were placed on 5 ml of the same R2/MS medium as described above contained in a petri dish of 6 cm in diameter. At the same time, 100 to 150 mg (fresh weight) of cultured cells of rice (Oryza sativa) was added to the petri dish.

These cultured rice cells were prepared in the following manner: A callus derived from a root of rice plant in the seedling stage was serially transferred once a week and cultured in a liquid medium. Small cell aggregates of not more than 1 mm in diameter which were present in the thus prepared suspension and showed active cell division were employed as the cultured rice cells.

The cultivation of the fused cells were effected under the dark at 25°C with slow shaking at about 30 rpm. The first division of these cells started to occur on the 3rd to 5th day of the cultivation and had finished by the 10th day. On the 10th day of cultivation, the cultured rice cells were removed and the agarose gel was transferred to another petri dish containing 5 ml of fresh R2/MS medium. The cultivation was further effected under the same conditions. After 3 to 4 weeks, colonies of 0.5 to 1.0 mm in diameter were formed. The number of colonies formed was approximately 5 to 10 times as large as that attained in the case of conventional cell fusion by PEG-DMSO technique.

These colonies were cultivated in N$_6$ soft agar medium (N$_6$: 2 mg/l 2,4-D, 0.5 mg/l benzylaminopurine, 6% sucrose, 0.25% agarose, pH 5.6) at 25°C under 3,000 lux for 2 to 3 weeks. Each colony was then transferred to N$_6$ growth medium (N$_6$: 2 mg/l 2,4-D, 0.5 mg/l benzylaminopurine, 6% sucrose, 0.5% agarose, pH 5.6), and further cultivated under the same conditions. A callus of 2 to 3 mm in diameter was formed in 2 to 3 weeks.

This callus was further cultivated in N$_6$ generation medium (N$_6$: 6% sucrose, pH 5.6) under the same conditions. After 2 to 8 weeks there was observed the formation of a somatic embryo. The somatic embryo was further grown by conventional procedures. A plant was thus obtained.

The thus obtained calluses were subjected to the conventional hybrid assay by analyzing the number of chromosomes, karyotypes, electrophoretic patterns of isozymes such as leucine aminopepridase, and morphological characteristics.

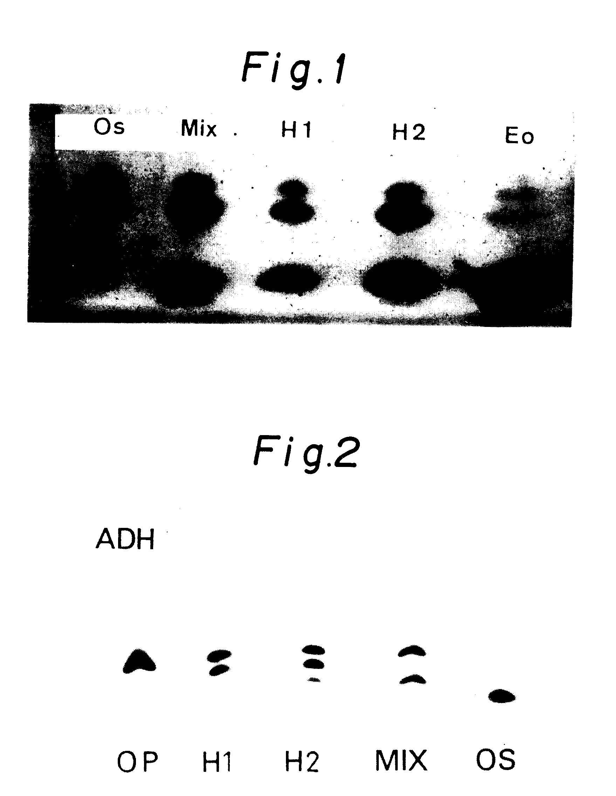

At least 50% of the obtained calluses were shown to be hybrid. Four calluses (H1 to H4) has the number of chromosomes as shown in Table 1 below. The electrophoretic patterns of isozymes for H1 and H2 are shown in Fig. 1.

Table 1

| Number of Chromosomes of Rice, Echinocloa and Hybrid Calluses | | | | | | |
|---|---|---|---|---|---|---|
| | Oryza sativa | Echinocloa oryzicola | Hybrid callus | | | |
| | | | H1 | H2 | H3 | H4 |
| Number of chromosomes | 24 | 36 | 120-130 | 120-130 | about 100 | about 120 |

EXAMPLE 2:

In the procedures of Example 1 (1), a seed of Oryza perrieri was used instead of the seed of Echinocloa oryzicola. The seed was treated for 4 to 5 hours at 45°C, sterilized, placed on MS agar medium (4 mg/l 2,4-D, 8 g/l Bactoagar, pH 5.8), and further treated in a similar manner to that of Example 1 to prepare protoplasts.

Then, the protoplast was fused with the protoplast from a cultivated rice Oryza sativa prepared in Example 1 (1) and (2), by the same method as in Example 1 (3).

The resulting fused cells were cultivated as in Example 1 (4) to produce plants H1 and H2. The electrophoretic results of their alcohol dehydrogenase (ADH) isozymes are shown in Fig. 2.

In the same procedures as in Example 1 (4), H1 and H2 were allowed to re-differentiate and then to grow to give a plant. Hybrid assay was performed based on the number of chromosomes, electrophoretic

EP 0 263 017 B1

pattern of isozymes, and morphological characteristics.

Further, hybrid plants between rice (Oryza sativa) and Oryza officinalis, Oryza eichingeri, or Oryza brachyantha were obtained by similar procedures.

As seen from the above examples, gramineous hybrid plants which cannot be obtained the conventional breeding methods may be prepared with a high reproducibility and a high efficiency according to the present invention.

**Claims**

**1.** A process for preparing a gramineous hybrid plant, said process comprising preparing a protoplast from a callus or suspension cell derived from Oryza sativa, preparing a protoplast from a callus or suspension cell derived from another gramineous plant, subjecting the protoplasts to cell fusion, characterised in that the fusion is effected by an electric treatment, said electric treatment comprising the application of direct current between successive alternating current applications, and cultivating the resulting fused cells in a culture medium containing cultured cells of Oryza sativa as nurse cells, and further cultivating the formed colonies in a culture medium containing a plant hormone

**2.** Process according to claim 1 characterised in that the electric treatment includes two successive applications of alternating current.

**3.** Process according to claim 1 or 2 characterised in that an alternating current of 2,000-7,000 kHz and 50-500 V/cm is applied.

**4.** Process according to claims 2 and 3 characterised in that the first application of alternating current has a duration of between 1 to 30 seconds, and the second application has a duration of between 1 to 200 seconds.

**5.** The process of any one of preceding claims, wherein the other gramineous plant is Echinocloa oryzicola.

**6.** The process according to any one of claims 1 to 5, wherein the other gramineous plant is Oriza perrieri, Oriza officinalis, Oriza eichingeri, or Oryza brachyantha.

**7.** The process according to any of claims 1 to 6, wherein prior to the cell fusion, the protoplast prepared from a callus or suspension cell derived from Oriza sativa is treated with an iodine compound while the protoplast prepared from a callus or suspension cell derived from another gramineous plant may optionally be treated with a Rhodamine compound.

**8.** The process according to any of claims 1 to 7, wherein the fused cells are co-cultivated in a culture medium containing cultured cells of Oryza sativa and 0.2 to 0.5 % of potassium nitrate as a nitrogen source to form colonies.

**9.** The process according to any of claims 1 to 8, wherein the plant hormone is 2.4-dichlorophenoxyacetic acid.

**Revendications**

**1.** Un procédé pour préparer une graminée hybride, ledit procédé comprenant la préparation d'un protoplaste à partir d'un cal ou d'une suspension de cellules dérivées d'Oryza sativa, la préparation d'un protopolaste à partir d'un cal ou d'une suspension de cellules dérivées d'une autre graminée, la soumission des protoplastes à une fusion cellulaire, caractérisé en ce que la fusion est effectuée par un traitement électrique, ledit traitement électrique comprenant l'application de courant continu entre des applications successives de courant alternatif, et on cultive les cellules fusionnées obtenues dans un milieu de culture contenant des cellules cultivées d'Oryza sativa comme cellules nourricières, et de plus on cultive les colonies formées dans un milieu de culture contenant une hormone végétale.

**2.** Procédé selon la revendication 1, caractérisé en ce que le traitement électrique comprend deux applications successives de courant alternatif.

6

**3.** Procédé selon la revendication 1 ou 2, caractérisé en ce que l'on applique un courant alternatif de 2 000 à 7 000 kHz et de 50 à 500 V/cm.

**4.** Procédé selon les revendications 2 et 3, caractérisé par une première application de courant alternatif pendant une durée de 1 à 30 secondes et une seconde application pendant une durée de 1 à 200 secondes.

**5.** Le procédé selon l'une quelconque des revendications précédentes, dans lequel l'autre graminée est Echinocloa oryzicola.

**6.** Le procédé selon l'une quelconque des revendications 1 à 5, dans lequel l'autre graminée est Oryza perrieri, Oryza officinalis, Oryza eichingeri ou Oryza brachyantha.

**7.** Le procédé selon l'une quelconque des revendications 1 à 6, dans lequel, avant la fusion cellulaire, on traite le protoplaste préparé à partir d'un cal ou d'une suspension de cellules dérivées d'Oryza sativa avec un composé iodé, tandis que l'on peut facultativement traiter le protoplaste préparé à partir d'un cal ou d'une suspension de cellules dérivées d'une autre graminée avec un composé de type Rhodamine.

**8.** Le procédé selon l'une quelconque des revendications 1 à 7, dans lequel les cellules fusionnées sont cocultivées dans un milieu de culture contenant des cellules cultivées d'Oryza sativa et 0,2 à 0,5 % de nitrate de potassium comme source d'azote pour former des colonies.

**9.** Le procédé selon l'une quelconque des revendications 1 à 8, dans lequel l'hormone végétale est l'acide 2,4-dichlorophénoxyacétique.

**Patentansprüche**

**1.** Verfahren zur Herstellung einer grasartigen Hybridpflanze durch Herstellen eines Protoplasten aus einem Callus oder einer Suspensionszelle abgeleitet von Oryza sativa. Herstellen eines Protoplasten aus einem Callus oder einer Suspensionszelle abgeleitet von einer andere grasartigen Pflanze und Unterwerfen der Protoplasten einer Zellfusion, **dadurch gekennzeichnet,** daß die Fusion durch eine elektrische Behandlung erfolgt, welche elektrische Behandlung das Anlegen eines Gleichstroms zwischen aufeinanderfolgendem Anlegen von Wechselstrom umfaßt, und die erhaltenen verschmolzenen Zellen in einem Kulturmedium züchtet, welches gezüchtete Zellen von Oryza sativa als Nährzellen enthält, und die gebildeten Kolonien weiter in einem Kulturmedium züchtet, welches ein Pflanzenhormon enthält.

**2.** Verfahren nach Anspruch 1, **dadurch gekennzeichnet,** daß die elektrische Behandlung zwei aufeinanderfolgende Anwendungen von Wechselstrom umfaßt.

**3.** Verfahren nach Anspruch 1 oder 2, **dadurch gekennzeichnet,** daß ein Wechselstrom von 2.000-7.000 kHz und 50-500 V/cm angewandt wird.

**4.** Verfahren nach den Ansprüchen 2 und 3, **dadurch gekennzeichnet,** daß die erste Anwendung des Wechselstroms eine Dauer zwischen 1 und 30 Sekunden und die zweite Anwendung eine Dauer zwischen 1 und 200 Sekunden umfaßt.

**5.** Verfahren nach einem dervorhergehenden Ansprüche, worin die andere grasartige Pflanze Echinocloa oryzicola ist.

**6.** Verfahren nach einem der Ansprüche 1 bis 5, worin die andere grasartige Pflanze Oriza perrieri, Oriza officinalis, Oriza eichingeri oder Oryza brachyantha ist.

**7.** Verfahren nach einem der Ansprüche 1 bis 6, worin der aus einem Callus oder einer Suspensionszelle, abgeleitet von Oriza sativa hergestellte Protoplast vor der Zellfusion mit einer Iodverbindung behandelt wird, während der aus einem Callus oder einer Suspensionszelle, die von einer anderen grasartigen Pflanze abgeleitet worden ist, hergestellte Protoplast gegebenenfalls mit einer Rhodaminverbindung behandelt werden kann.

8. Verfahren nach einem der Ansprüche 1 bis 7, worin die verschmolzenen Zellen gemeinsam in einem Kulturmedium, welches gezüchtete Zellen von Oryza sativa und 0,2 bis 0,5 % Kaliumnitrat als Stickstoffquelle enthält, gemeinsam zur Bildung von Kolonien gezüchtet werden.

9. Verfahren nach einem der Ansprüche 1 bis 8, worin das Pflanzenhormon 2,4-Dichlorphenoxyessigsäure ist.

# Fig.1

Os    Mix    H1    H2    Eo

# Fig.2

ADH

OP    H1    H2    MIX    OS